# NEW EUROPEAN PATENT SPECIFICATION

(11) **EP 3 043 768 B2**
(45) Date of publication and mention of the opposition decision: **29.06.2022**
(45) Mention of the grant of the patent: 01.05.2019
(21) Application number: 13753523.3
(22) Date of filing: 13.08.2013
(51) Int. Cl.: A61K 8/365, A61K 8/368, A61K 8/55, A61Q 5/06, A61K 8/73

(54) **HAIR TREATING METHOD**
HAARBEHANDLUNGSMETHODE
MÉTHODE COSMÉTIQUE DE TRAITEMENT DES CHEVEUX

(43) Date of publication of application: 20.07.2016
(73) Proprietor: L'OREAL, 75008 Paris (FR); Biato, Camila, 22795-295 Rio de Janeiro (BR)
(72) Inventor: BIATO, Camila, 22795-295 Rio de Janeiro (BR)
(74) Representative: Lavoix
(86) International application number: PCT/BR2013/000305
(87) International publication number: WO 2015/021517

(56) References cited:
- EP-A2- 2 153 818
- WO-A2-2013/076061
- US-A1- 2006 045 862
- US-A1- 2006 150 344
- DATABASE GNPD [Online] MINTEL; 1 October 2011 (2011-10-01), "Quick blow dry", XP002724047, Database accession no. 1655457
- DATABASE GNPD [Online] MINTEL; 1 July 2007 (2007-07-01), "Twist Around Lotion", XP002724048, Database accession no. 740297
- DATABASE GNPD [Online] MINTEL; 1 September 2009 (2009-09-01), "Glossing Straigthener", XP002724049, Database accession no. 1172479
- DATABASE GNPD MINTEL; 1 July 2008 (2008-07-01), "Iron Styler", XP002724050, Database accession no. 941223
- Christina Patrice: "How to Straighten Your Hair and Prevent Heat Damage [2013 Edition]", The Mane Objective , 24 May 2013 (2013-05-24), XP002724044, Retrieved from the Internet: URL:http://www.maneobjective.com/2013/05/h ow-to-straighten-your-hair-and-prevent.htm l [retrieved on 2014-04-22]
- DATABASE GNPD [Online] MINTEL; 1 August 2004 (2004-08-01), "Hydra Finalizing Anti Frizz Gel Creme", XP002724052, Database accession no. 10183950
- WORTMANN FRANZ J ET AL: "Thermal denaturation and structural changes of [alpha]-helical proteins in keratins", JOURNAL OF STRUCTURAL BIOLOGY, vol. 177, no. 2, 19 October 2011 (2011-10-19), pages 553-560, XP028891902, ISSN: 1047-8477, DOI: 10.1016/J.JSB.2011.09.014
- DATABASE GNPD [Online] MINTEL; 1 April 2013 (2013-04-01), "Damaged Hair S.O.S. Double Serum", Database accession no. 2044609
- DATABASE GNPD [Online] MINTEL; 1 April 2006 (2006-04-01), "Oil Therapy", Database accession no. 10255135
- DATABASE GNPD [Online] MINTEL; 1 January 2013 (2013-01-01), "Heat Tamer Leave-In Spray", Database accession no. 1967245
- "Ingredient list of ApHogee Restructurizer", , 14 February 2018 (2018-02-14), Retrieved from the Internet: URL:https://www.amazon.com/ApHogee-Keratin -Green-Tea-Restructurizer/dp/B000142P12?th =1 [retrieved on 2018-02-14]
- DATABASE GNPD October 2012 (2012-10), "Luxury Keratin Treatment", retrieved from Mintel Database accession no. 1892615

## Description

The present invention relates to a process for treating hair with a composition comprising an organic monoacid and a cellulosic polymer.

In the hair field, consumers wish to have available compositions which make it possible to introduce a temporary change to their hair and to do this while targeting a good wear property for the effect produced. In general, it is desirable for the change to withstand shampooing operations for a minimum of 15 days, indeed even more, depending on the nature of the said change.

Treatments already exist for modifying the colour or the shape of the hair and also, to some extent, the texture of the hair. One of the known treatments for modifying the texture of the hair consists of the combination of heat and of a formaldehyde-comprising composition. This treatment is especially effective in conferring a better appearance on damaged hair and/or in treating long hair and curly hair.

The heat use can be that of the iron (flat tongs or crimping iron), the temperature of which can generally reach 200°C or more.

However, there is an increasing desire to avoid the use of such substances, which can prove to be aggressive to the hair and other keratinous substances.

Application WO2011/104282 has thus provided a novel method for semi-permanently smoothing the hair which consists in applying an α-aceto acid solution to the hair for from 15 to 120 minutes, in then drying and, finally, in smoothing the hair with an iron at a temperature of approximately 200°C. The α-aceto acid employed is preferably glyoxylic acid.

However, it has been found that glyoxylic acid may not always be well tolerated, in particular when the scalp is sensitive and/or irritated. Its volatility combined with a hot process may cause problems.

Furthermore, cosmetic formulations of prior art can detrimentally affect the hair and/or detrimentally affect the colour thereof. In point of fact, the desired treatment is very particularly intended to repair artificially dyed hair. It is therefore preferable to avoid the use of active agents capable of introducing a reinforcing effect but while contributing to detrimentally affecting the hair and/or its artificial colour.

The object of the invention is to develop a method for changing the aspect of the hair, such as smoothing and/or straightening hair without detrimental effect on the integrity and/or color of the hair fibres. Another object of the invention is to develop a method for changing the aspect of the hair and resistant to water.

Thus, a subject-matter of the present invention is a method for the treatment of hair, as defined in claim 1.

It has been found that the method according to the invention makes it possible to obtain a solution for treatment of the hair in a way which is lasting and compatible with any type of past or future hair treatment.

In particular, the method according to the invention will not detrimentally affect the integrity of the fibre. In addition, it will improve qualities of use and the mechanical properties of the hair, such as the resistance to moisture, and the sheen, and will reduce volume and frizz. It will also reduce colour modification of hair.

In that which follows, the expression "at least one" is equivalent to the expression "one or more".

The method of the present invention is carried out with a composition comprising glycolic acid with at least one additional monocarboxylic acid of formula (I), their stereoisomers, their organic or mineral salts or their corresponding solvates: 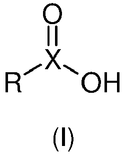 wherein X represents a carbon atom and R represents an alkyl, alkenyl, aryl or aralkyl group with a linear, branched or cyclic alkyl group comprising from 1 to 20 carbon atoms, a linear, branched or cyclic alkenyl group comprising from 2 to 20 carbon atoms, an aryl or aralkyl group comprising from 6 to 20 carbon atoms, these groups being substituted by one or more groups selected from halogen atoms and from the hydroxyl, trifluoromethyl, and alkoxy C1-C4 groups.

Among the monoacid of formula (I), mention can be made of lactic acid, 1-hydroxy-1-cyclopropanecarboxylic acid, 2-hydroxy-3-butenoic acid, 2-hydroxyisobutyric acid, 3-hydroxybutyric acid, 4-hydroxybutyric acid, 2-hydroxy-n-butyric acid, glyceric acid, 2-hydroxy-3-methylbutyric acid, 2-hydroxy-2-methylbutyric acid, 2-hydroxypentanoic acid, 3-hydroxy pentanoic acid, 4-hydroxypentanoic acid, 2-hydroxyvaleric acid, 1-hydroxycyclohexanecarboxylic acid, mandelic acid, 2-hydroxy-3-methylvaleric acid, 2-trifluoromethyl-2-hydroxypropionic acid, hexahydroxymandelic acid, 2-hydroxyoctanoic acid, 3-phenylactic acid, 3-hydroxymandelic acid, 4-hydroxymandelic acid, 2-hydroxynonanoic acid, 3-methoxymandelic acid, 4-methoxymandelic acid, 3-(4-hydroxyphenyl)lactic acid, 1-cyclopentanol-1-carboxylic acid, 1,2-dihydroxycyclobutanecarboxylic acid, 2-ethyl-2-hydroxybutyric acid, 2-hydroxy-3,3-dimethylbutyric acid, gluconic acid, salicylic acid, 2-hydroxyhexanoic acid, 3-hydroxyhexanoic acid, 4-hydroxyhexanoic acid, 5-hydroxyhexanoic acid, 6-hydroxyhexanoic acid, and the stereoisomers, organic or mineral salts and solvates thereof.

Particularly preferred monoacid of formula (I) are selected from: lactic acid, glyceric acid, gluconic acid, salicylic acid, 2-hydroxyisobutyric acid, 3-hydroxybutyric acid, 4-hydroxybutyric acid, 2-hydroxy-n-butyric acid, 2-hydroxyhexanoic acid, 3-hydroxyhexanoic acid, 4-hydroxyhexanoic acid, 5-hydroxyhexanoic acid, 6-hydroxyhexanoic acid, 2-hydroxypentanoic acid, 3-hydroxypentanoic acid, 4-hydroxypentanoic acid, their stereoisomers, their organic or mineral salts or their corresponding solvates.

The moncarboxylic acids are substituted on R radical by at least one hydroxyl group. The R radical is an alkyl, alkenyl, aryl or aralkyl group with a linear, branched or cyclic alkyl group comprising from 1 to 20 carbon atoms, a linear, branched or cyclic alkenyl group comprising from 2 to 20 carbon atoms, an aryl or aralkyl group comprising from 6 to 20 carbon atoms, these groups being substituted by at least one hydroxyl group being preferably located in position alpha or beta from the carboxy group and these groups being eventually substituted by one or more other groups selected from halogen atoms and from the groups trifluoromethyl, and alkoxy C1-C4.

According to this preference, monoacids of formula (I) are selected from lactic acid, glyceric acid, gluconic acid, salicylic acid, 2-hydroxyisobutyric acid, 2-hydroxy-n-butyric acid, 2-hydroxyhexanoic acid, 2-hydroxypentanoic acid their stereoisomers, their organic or mineral salts or their corresponding solvates.

More preferably monoacids of formula (I) are selected from lactic acid, glyceric acid, gluconic acid, salicylic acid, their stereoisomers, their organic or mineral salts or their corresponding solvates.

The method of the invention is carried out with a composition comprising at least glycolic acid with at least one additional monocarboxylic acid of formula (I). Preferably, the composition contains glycolic acid and salicylic acid.

The amount of monoacids, their stereoisomers, their organic or mineral salts or their corresponding solvates in the composition useful in the method of the present invention varies largely. However, monoacids, their stereoisomers, their organic or mineral salts or their corresponding solvates are preferably present in the composition at a concentration ranging from 0.01% to 50% by weight, in particular from 0.1% to 20% by weight and preferably from 1% to 5% by weight, with respect to the total weight of the composition.

The composition useful in the method of the invention contains a cellulosic polymer as defined in claim 1.

The term *"cellulose"* polymer means, according to the invention, any polysaccharide compound having in its structure sequences of glucose residues linked together via β-1,4 linkages; in addition to unsubstituted celluloses, the cellulose derivatives may be anionic, cationic, amphoteric or non-ionic. Thus, the cellulose polymers of the invention can be chosen from unsubstituted celluloses, including in a microcrystalline form, and cellulose ethers. Among these cellulose polymers, cellulose ethers, cellulose esters and cellulose ester ethers are distinguished. Among the cellulose esters are inorganic esters of cellulose (cellulose nitrates, sulfates, phosphates, etc.), organic cellulose esters (cellulose monoacetates, triacetates, amidopropionates, acetatebutyrates, acetatepropionates and acetatetrimellitates, etc.), and mixed organic/inorganic esters of cellulose, such as cellulose acetatebutyrate sulfates and cellulose acetatepropionate sulfates. Among the cellulose ester ethers, mention may be made of hydroxypropylmethylcellulose phthalates and ethylcellulose sulfates.

According to the invention, the cellulose polymer(s) are non-associative.

The *"non-associative"*cellulose polymers of the invention are cellulose polymers which do not comprise a fatty chain, i.e. which preferably do not comprise a C₁₀-C₃₀ chain in their structure.

The cellulose polymer(s) are non-ionic. Mention may be made of (C₁-C₄)alkylcelluloses such as methylcelluloses and ethylcelluloses for example Ethocel standard 100 Premium from Dow Chemical; (poly)hydroxy(C₁-C₄)alkylcelluloses such as hydroxymethylcelluloses, hydroxyethylcelluloses for example Natrosol 250 HHR provided by Aqualon and hydroxypropylcelluloses for example Klucel EF from Aqualon; mixed (poly)hydroxy(C₁-C₄)alkyl-(C₁-C₄)alkylcelluloses such as hydroxypropylmethylcelluloses for example Methocel E4M from Dow Chemical; hydroxyethylmethylcelluloses, hydroxyethylethylcelluloses for example Bermocoll E 481 FQ from Akzo Noble and hydroxybutylmethylcelluloses.

The cellulose polymers are non-ionic and free of fatty chain (non associative non ionic alkylcellulose polymer). The cellulose polymer(s) useful in the invention are chosen from non ionic cellulose ethers selected from (C₁-C₄)alkyl-celluloses, (poly)hydroxy(C₁-C₄)alkylcelluloses; (poly)hydroxy(C₁-C₄)alkyl-(C₁-C₄)alkylcelluloses such as hydroxypropylmethylcelluloses; hydroxyethylmethylcelluloses; hydroxyethylethylcelluloses; hydroxybutylmethylcelluloses; hydroxymethylcelluloses; hydroxyethylcelluloses and hydroxypropylcelluloses.

The cellulose polymer(s) may be present in the composition useful in the invention in an amount ranging from 0.05% to 10% by weight, in particular from 0.1% to 5% by weight and preferably from 0.2% to 3% by weight relative to the total weight of the composition.

The composition used in the method of the invention preferably does not contain a colouring agent or reducing agent.

The term "colouring agents" is understood to mean, according to the present invention, agents for colouring keratinous fibres, such as direct dyes, pigments or oxidation dye precursors (bases and couplers). If they are present, their content does not exceed 0.001% by weight, with respect to the total weight of the composition. This is because, at such content, only the composition would be dyed, that is to say that an effect of dyeing the keratinous fibres would not be observed.

It should be remembered that oxidation dye precursors, oxidation bases and couplers are colourless or only slightly coloured compounds which, by a condensation reaction in the presence of an oxidizing agent, give a coloured entity. With regard to direct dyes, these compounds are coloured and exhibit a degree of affinity for the keratinous fibres.

The term "reducing agent" is understood to mean, according to the present invention, an agent capable of reducing the disulfide bonds of the hair, such as the compounds chosen from thiols, alkali metal sulfites, hydrides or phosphines.

The compositions used according to the invention can be provided in any formulation form conventionally used and in particular in the form of an aqueous, alcoholic or aqueous/alcoholic solution or suspension or oily solution or suspension; of a solution or a dispersion of the lotion or serum type; of an emulsion, in particular having a liquid or semiliquid consistency, of the O/W, W/O or multiple type; of a suspension or emulsion having a soft consistency of (O/W) or (W/O) cream type; of an aqueous or anhydrous gel, or of any other cosmetic form.

The composition can comprise one or more organic solvents, for example water-soluble solvents such as C₁₋C₇ alcohols; mention may be made of aliphatic C₁₋C₇ monoalcohols, aromatic C₆-C₇ monoalcohols, C₃-C₇ polyols or C₃-C₇ polyol ethers, which can be employed alone or as a mixture with water.

The compositions of the invention can be aqueous or anhydrous. Advantageously, the composition comprises from 20 to 95% of water based on the total weight of the composition.

More preferably the composition is a hydro alcoholic composition

The composition used in the method of the invention can additionally comprise at least one additional ingredient for example chosen from oils; solid fatty substances such as fatty alcohol, fatty ester; non-ionic surfactants, surfactants ; sunscreens ; moisturizing agents; antidandruff agents; antioxidants; chelating agents; pearlescent and opacifying agents; plasticizing or coalescing agents; fillers; silicones an particularly aminosilicones; thickeners other than cellulosic polymers ; gelling agents ; emulsifiers ; conditioning or styling polymers; fragrances; basifying or acidifying agents; silanes; or crosslinking agents.

Depending on their nature and the purpose of the composition, the additional ingredients can be present in an amount which can be easily determined by a person skilled in the art.

In the method of the invention, the composition exhibits preferably an acidic pH, more preferably less than 4.In a preferred embodiment the pH can vary from 1 to 3, preferably from 1,5 to 3, better from 1,7 to 3.

pH can be adjusted with acidic agents other than organic monoacids of the invention such as mineral acids as chlorhydric acid or phosphoric acid, or with basic agents such as mineral basic agents as ammonia,carbonates, bicarbonates, hydroxides or organic basic agents such as alcanolamines.

These compositions can be packaged in pump-action sprays or in aerosol containers, in order to provide for application of the composition in the vaporized (lacquer) form or in the form of a mousse. Such packaging forms are indicated, for example, when it is desired to obtain a spray or a mousse, for the treatment of the hair. In these cases, the composition preferably comprises at least one propellant.

The method according to the invention can be provided in the form of a hair product, in particular a hair care, styling or shaping product. The method especially finds advantageous applications for the retention of the hairstyle or the shaping of the hair, or the volume and frizz reduction. Additional advantages are for the conditioning, shaping, reinforcing and/or repairing of the hair, in particular for improving the disentangling, smoothing, combability, manageability and softness of the hair.

The method of the invention may comprise a heating step at a temperature higher than 150°C, preferably ranging from 150 to 250°C. The heating step can be carried out with irons such as flat irons, flat tongs or a crimping iron capable of raising the temperature to a value ranging from 150°C to 250°C.

According to a specific embodiment, the composition is applied on dried hair. After a posing time, usually of at least 10 min, preferably between 15 and 45 min, more preferably around 30 min, the hair are brushed and then straightened with a flat iron at a temperature of at least 150°C, preferably ranging from 200 to 250°C. Then the hair are rinsed with water and dried.

Before the application of the composition, the hair can be shampooed and dried.

The method of the invention is preferably used without a deformation step using reducing agent or a composition with pH over 10.

The following examples serve to illustrate the invention without, however, exhibiting a limiting nature.

### EXAMPLES

The following composition was prepared at the time of use (given in weight %), pH of the composition being adjusted to 2.2 ± 0.2 :

| | |
|---|---|
| METHYL HYDROXYETHYLCELLULOSE sold under the name Structure ^{®}cell 8000 M by AKZONOBEL | 1% |
| GLYCOLIC ACID in aqueous solution (70 %) sold under the name GLYPURE 70 by DUPONT | 10% |
| SALICYLIC ACID sold by Novacyl | 2% |
| ETHANOL | 30% |
| SODIUM HYDROXIDE | 0.027% |
| AMODIMETHICONE (and) TRIDECETH-5 (and) TRIDECETH-10 sold under the name DC(R)2-2899 by DOW CORNING | 2% |
| AMODIMETHICONE (and) TRIDECETH-6 (and) CETRIMONIUM CHLORIDE sold under the name Belsil ADM by Wacker | 2% |
| WATER | QSP |

The above composition was applied on shampooed and blow-dried naturally curled hair. After having left the composition on hair for 30 min, the hair were brushed and then heated with a flat iron at a temperature from 200-250°C. Then the hair was rinsed.

By using the method of the invention, the hair thus treated exhibit a substantial reduction of volume.

## Claims

1. Method for the treatment of hair comprising the application to the hair of a composition, comprising at least glycolic acid with at least one additional monocarboxylic acid of formula (I), their stereoisomers, their organic or mineral salts or their corresponding solvates, and at least one cellulosic polymer, and a step of heating the hair at a temperature higher than 100°C, 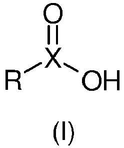
wherein X is a carbon atom and R radical is an alkyl, alkenyl, aryl or aralkyl group with a linear, branched or cyclic alkyl group comprising from 1 to 20 carbon atoms, a linear, branched or cyclic alkenyl group comprising from 2 to 20 carbon atoms, an aryl or aralkyl group comprising from 6 to 20 carbon atoms, these groups being substituted by at least one hydroxyl group being preferably located in position alpha or beta from the carboxy group and these groups being optionally substituted by one or more other groups selected from halogen atoms and from the groups trifluoromethyl, and alkoxy C1-C4,
and wherein said cellulosic polymer selected from non associative non ionic cellulosic polymer selected from (C₁-C₄)alkylcelluloses, (poly)hydroxy(C₁-C₄)alkylcelluloses; and (poly)hydroxy(C₁-C₄)alkyl-(C₁-C₄)alkylcelluloses.

2. Method according to claim 1, wherein the monocarboxylic acids are selected from lactic acid, glyceric acid, gluconic acid, salicylic acid, 2-hydroxyisobutyric acid, 2-hydroxy-n-butyric acid, 2-hydroxyhexanoic acid, 2-hydroxypentanoic acid, their stereoisomers, their organic or mineral salts or their corresponding solvates, and more preferably are selected from lactic acid, glyceric acid, gluconic acid, salicylic acid, their stereoisomers, their organic or mineral salts or their corresponding solvates.

3. Method according to any of the preceding claims, wherein the composition comprises glycolic acid and salicylic acid.

4. Method according to any of the preceding claims, wherein the monoacids, their stereoisomers, their organic or mineral salts or their corresponding solvates are present in the composition in an amount from 0.1 to 50% by weight, in particular from 0.1% to 20% by weight, and preferably from 1% to 15% by weight, with respect to the total weight of the composition.

5. Method according to any of the preceding claims, wherein the cellulosic polymer is selected from hydroxypropylmethylcelluloses; hydroxyethylmethylcelluloses; hydroxyethylethylcelluloses; hydroxybutylmethylcelluloses; hydroxymethylcelluloses; hydroxyethylcelluloses; and hydroxypropylcelluloses.

6. Method according to any of the preceding claims, wherein the cellulosic polymer(s) are present in an amount ranging from 0.05% to 10% by weight, in particular from 0.1% to 5% by weight, and preferably from 0.2% to 3% by weight, relative to the total weight of the composition.

7. Method according to any of the preceding claims, wherein the composition is hydro alcoholic.

8. Method according to any of the preceding claims, wherein the composition exhibits an acidic pH, preferably less than 4, preferably varying from 1 to 3, more preferably from 1.5 to 3, better from 1.7 to 3.

9. Method according to one of the preceding claims, wherein the heating step is carried out at a temperature from 150 to 250°C, preferably with flat irons.

10. Method according to any one of the preceding claims, wherein the composition is applied on dried hair, and after a posing time of at least 10 min, preferably from 15 to 45 min, the hair are brushed and then straightened with a flat iron at a temperature of at least 150°C, preferably ranging from 200 to 250°C.

## Patentansprüche

1. Verfahren zur Behandlung von Haar, umfassend das Auftragen einer Zusammensetzung auf das Haar, umfassend mindestens Glykolsäure mit mindestens einer weiteren Monocarbonsäure von Formel (I), ihre Stereoisomere, ihre organischen oder mineralischen Salze oder ihre entsprechenden Solvate und mindestens ein Cellulosepolymer, und einen Schritt eines Erhitzens des Haars auf eine Temperatur von über 100 °C, 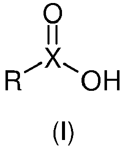
worin X ein Kohlenstoffatom ist und R-Radikal eine Alkyl-, Alkenyl-, Aryl- oder Aralkylgruppe mit einer linearen, verzweigten oder cyclischen Alkylgruppe umfassend 1 bis 20 Kohlenstoffatome, einer linearen, verzweigten oder cyclischen Alkenylgruppe umfassend 2 bis 20 Kohlenstoffatome, einer Aryl- oder Aralkylgruppe umfassend 6 bis 20 Kohlenstoffatome ist, wobei diese Gruppen durch mindestens eine Hydroxylgruppe substituiert sind, die vorzugsweise an alpha- oder beta-Position zu der Carboxygruppe ist, und wobei diese Gruppen optional durch eine oder mehrere andere Gruppen substituiert sind, ausgewählt aus Halogenatomen und aus den Gruppen Trifluormethyl und Alkoxy C1-C4,
und wobei das Cellulosepolymer ausgewählt ist aus nicht-assoziativen, nicht-ionischen Cellulosepolymeren, ausgewählt aus (C₁-C₄)-Alkylcellulosen, (Poly)hydroxy-(C₁-C₄)-Alkylcellulosen und (Poly)hydroxy-(C₁-C₄)-Alkyl-(C₁-C₄)-Alkylcellulosen.

2. Verfahren nach Anspruch 1, wobei die Monocarbonsäuren ausgewählt sind aus Milchsäure, Glycerinsäure, Gluconsäure, Salicylsäure, 2-Hydroxyisobuttersäure, 2-Hydroxy-n-buttersäure, 2-Hydroxyhexansäure, 2-Hydroxypentansäure, deren Stereoisomeren, ihren organischen oder anorganischen Salzen oder ihren entsprechenden Solvaten und bevorzugter ausgewählt sind aus Milchsäure, Glycerinsäure, Gluconsäure, Salicylsäure, ihren Stereoisomeren, ihre organischen oder anorganischen Salzen oder ihren entsprechenden Solvaten.

3. Verfahren nach einem der vorherigen Ansprüche, wobei die Zusammensetzung Glykolsäure und Salicylsäure umfasst.

4. Verfahren nach einem der vorherigen Ansprüche, **dadurch gekennzeichnet, dass** die Monosäuren, ihre Stereoisomere, ihre organischen oder mineralischen Salze oder ihre entsprechenden Solvate in der Zusammensetzung in einer Menge von 0,1 bis 50 Gewichtsprozent, insbesondere von 0,1 bis 20 Gewichtsprozent und vorzugsweise von 1 bis 15 Gewichtsprozent, bezogen auf das Gesamtgewicht der Zusammensetzung, vorhanden sind.

5. Verfahren nach einem der vorherigen Ansprüche, wobei das Cellulosepolymer ausgewählt ist aus Hydroxypropylmethylcellulosen, Hydroxyethylmethylcellulosen, Hydroxyethylethylcellulosen, Hydroxybutylmethylcellulosen, Hydroxymethylcellulosen, Hydroxyethylcellulosen und Hydroxypropylcellulosen.

6. Verfahren nach einem der vorherigen Ansprüche, wobei das/die Cellulosepolymer(e) in einer Menge von 0,05 bis 10 Gewichtsprozent, insbesondere von 0,1 bis 5 Gewichtsprozent und vorzugsweise von 0,2 bis 3 Gewichtsprozent, bezogen auf das Gesamtgewicht der Zusammensetzung, vorhanden sind.

7. Verfahren nach einem der vorherigen Ansprüche, wobei die Zusammensetzung hydro-alkoholisch ist.

8. Verfahren nach einem der vorherigen Ansprüche, wobei die Zusammensetzung einen sauren pH aufweist, vorzugsweise unter 4, vorzugsweise variierend von 1 bis 3, bevorzugter von 1,5 bis 3, besser von 1,7 bis 3.

9. Verfahren nach einem der vorherigen Ansprüche, wobei der Erhitzungsschritt bei einer Temperatur von 150 bis 250 °C, vorzugsweise mit Haarglätter, durchgeführt wird.

10. Verfahren nach einem der vorherigen Ansprüche, wobei die Zusammensetzung auf das getrocknete Haar aufgetragen wird und nach einer Einwirkzeit von mindestens 10 Minuten, vorzugsweise von 15 bis 45 Minuten, das Haar gebürstet und anschließend mit einem Haarglätter bei einer Temperatur von mindestens 150 °C, vorzugsweise im Bereich von 200 bis 250 °C, geglättet wird.

## Revendications

1. Procédé de traitement des cheveux comprenant l'application sur les cheveux d'une composition, comprenant au moins de l'acide glycolique avec au moins un acide monocarboxylique supplémentaire de la formule (I), leurs stéréoisomères, leurs sels organiques ou minéraux ou leurs solvates correspondants, et au moins un polymère cellulosique, et une étape de chauffage des cheveux à une température supérieure à 100 °C, 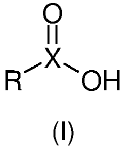
dans laquelle X représente un atome de carbone et le radical R représente un groupe alkyle, alcényle, aryle ou aralkyle avec un groupe alkyle linéaire, ramifié ou cyclique comprenant de 1 à 20 atomes de carbone, un groupe alcényle linéaire, ramifié ou cyclique comprenant de 2 à 20 atomes de carbone, un groupe aryle ou aralkyle comprenant de 6 à 20 atomes de carbone, ces groupes étant substitués par au moins un groupe hydroxyle situé de préférence en position alpha ou bêta du groupe carboxy et ces groupes étant éventuellement substitués par un ou plusieurs autres groupes choisis parmi des atomes d'halogène et les groupes trifluorométhyle et alcoxy en C1-C4,
et dans lequel ledit polymère cellulosique est choisi parmi les polymères cellulosiques non associatifs non ioniques choisis parmi les (C₁-C₄)alkylcelluloses, les (poly)hydroxy(C₁-C₄)alkylcelluloses ; et les (poly)hydroxy(C₁-C₄)alkyl-(C₁-C₄)alkylcelluloses.

2. Procédé selon la revendication 1, dans lequel les acides monocarboxyliques sont choisis parmi l'acide lactique, l'acide glycérique, l'acide gluconique, l'acide salicylique, l'acide 2-hydroxyisobutyrique, l'acide 2-hydroxy-n-butyrique, l'acide 2-hydroxyhexanoïque, l'acide 2-hydroxypentanoïque, leurs stéréoisomères, leurs sels organiques ou minéraux ou leurs solvates correspondants, et plus préférablement sont choisis parmi l'acide lactique, l'acide glycérique, l'acide gluconique, l'acide salicylique, leurs stéréoisomères, leurs sels organiques ou minéraux ou leurs solvates correspondants.

3. Procédé selon l'une quelconque des revendications précédentes, dans lequel la composition comprend de l'acide glycolique et de l'acide salicylique.

4. Procédé selon l'une quelconque des revendications précédentes, dans lequel les monoacides, leurs stéréoisomères, leurs sels organiques ou minéraux ou leurs solvates correspondants sont présents dans la composition dans une quantité comprise entre 0,1 et 50 % en poids, en particulier entre 0,1 % et 20 % en poids, et de préférence entre 1 % et 15 % en poids, par rapport au poids total de la composition.

5. Procédé selon l'une quelconque des revendications précédentes, dans lequel le polymère cellulosique est choisi parmi les hydroxypropylméthylcelluloses ; les hydroxyéthylméthylcelluloses ; les hydroxyéthyléthylcelluloses ; les hydroxybutylméthylcelluloses ; les hydroxyméthylcelluloses ; les hydroxyéthylcelluloses ; et les hydroxypropylcelluloses.

6. Procédé selon l'une quelconque des revendications précédentes, dans lequel le ou les polymères cellulosiques sont présents dans une quantité comprise entre 0,05% et 10% en poids, en particulier entre 0,1 % et 5 % en poids, et de préférence entre 0,2 % et 3 % en poids, par rapport au poids total de la composition.

7. Procédé selon l'une quelconque des revendications précédentes, dans lequel la composition est hydroalcoolique.

8. Procédé selon l'une quelconque des revendications précédentes, dans lequel la composition présente un pH acide, de préférence inférieur à 4, de manière plus préférable variant de 1 à 3, de manière encore plus préférable variant de 1,5 à 3, et de manière encore plus préférable variant de 1,7 à 3.

9. Procédé selon l'une des revendications précédentes, dans lequel l'étape de chauffage est réalisée à une température comprise entre 150 et 250 °C, de préférence avec des fers plats.

10. Procédé selon l'une quelconque des revendications précédentes, dans lequel la composition est appliquée sur des cheveux secs, et après un temps de pose d'au moins 10 min, de préférence entre 15 et 45 min, les cheveux sont brossés puis lissés au fer plat à une température d'au moins 150 °C, de préférence comprise entre 200 et 250 °C.
